# FASCICULE DE BREVET EUROPEEN

(11) **EP 1 558 094 B1**
(45) Date de publication et mention de la délivrance du brevet: **03.01.2007**
(21) Numéro de dépôt: 03778451.9
(22) Date de dépôt: 24.10.2003
(51) Int. Cl.: A23L 1/00, A23L 1/09, A61K 9/16

(54) **UTILISATION DE MALTODEXTRINES BRANCHEES COMME LIANTS DE GRANULATION**
VERWENDUNG VON VERZWEIGTEM MALTODEXTRIN ALS GRANULIERBINDEMITTEL
USE OF BRANCHED MALTO-DEXTRINS AS GRANULATION BINDERS

(30) Priorité: 06.11.2002 FR 0213868
(43) Date de publication de la demande: 03.08.2005
(73) Titulaire: Roquette Frères, 62136 Lestrem (FR)
(72) Inventeur: SERPELLONI, Michel, F-62660 Beuvry-les-Béthune (FR)
(74) Mandataire: Boulinguiez, Didier
(86) Numéro de dépôt international: PCT/FR2003/003156
(87) Numéro de publication internationale: WO 2004/043166

(56) Documents cités:
- EP-A- 1 006 128
- US-A- 3 974 032
- US-A- 5 458 892
- US-A- 5 612 202
- US-A- 5 886 168
- US-A1- 2002 146 487
- US-B1- 6 348 264

## Description

La présente invention est relative à la préparation de granules de substances actives renfermant des fibres alimentaires.

Plus particulièrement, l'invention est relative à un procédé de préparation de granules de substances actives renfermant des fibres alimentaires, qui consiste à granuler un mélange desdites substances actives avec des maltodextrines branchées, lesdites maltodextrines branchées représentant entre 3 et 13 % en poids du mélange à granuler. Elle vise également l'utilisation de ces maltodextrines branchées comme liants de granulation de substances actives.

Par « maltodextrines branchées », on entend au sens de la présente invention les maltodextrines décrites dans la demande de brevet EP 1.006.128 dont la société Demanderesse est titulaire.

Ces maltodextrines branchées présentent entre 15 et 35 % de liaisons glucosidiques 1-6, une teneur en sucres réducteurs inférieure à 20 %, un indice de polymolécularité inférieur à 5 et une masse moléculaire moyenne en nombre Mn au plus égale à 4500 g/mole.

Au sens de l'invention, on entend également par « substances actives » des composés qui présentent des intérêts alimentaires et/ou pharmaceutiques, tels que par exemple des composés de type amidons, dérivés d'amidon (tels les produits de l'hydrolyse acide ou enzymatique de l'amidon ou les produits d'hydrogénation desdits hydrolysats d'amidon), des sucres, mais également des composés de type édulcorants intenses, des enzymes, des vitamines ou des principes actifs pharmaceutiques, pris seuls ou en combinaison, qui ne présentent peu ou pas d'aptitude particulière à la granulation.

Des substances telles les protéines, notamment les protéines de soja, ne sont pas retenues ici car elles présentent naturellement des propriétés de coagulation classiquement mises à profit pour leur granulation.

Dans le domaine alimentaire, ces substances actives peuvent être plus particulièrement des polysaccharides ou des oligosaccharides de type dextrines, maltodextrines, polydextrose ou fructo-oligosaccharides, mais aussi des polyols, tels que par exemple le sorbitol, le xylitol, le mannitol, le lactitol, le maltitol, l'érythritol et l'isomalt.

Il est connu d'utiliser ces substances actives en tant que telles, mais également comme agent de charge pour d'autres substances actives tels les édulcorants intenses, préparés par synthèse chimique de type saccharine, aspartame, acésulfame K, cyclamate, stévioside, sucralose, néotame ou alitame.

Dans le domaine pharmaceutique, il peut être souhaité d'utiliser ces agents de charge en association avec par exemple des principes actifs pharmaceutiques tels les analgésiques antipyrétiques comme l'aspirine et ses dérivés, les dérivés du paraminophénol (tels que le paracétamol) ou les dérivés de la pyrazolone.

Les opérations de granulation sont souvent mises en oeuvre pour valoriser la forme poudre de ces substances actives ou ces mélanges de substances actives car, pour des raisons techniques et économiques, il est souvent recherché d'augmenter leur granulométrie.

Deux raisons principales sont données pour cette nouvelle formulation :
- la première est essentiellement commerciale, car le granule a une meilleure présentation ou est plus facile à manipuler par l'utilisateur,
- la deuxième raison est strictement technique, parce qu'un granule entre plus facilement dans un processus de fabrication rationnel, en termes de facilité de convoyage, de meilleur écoulement, d'absence de formation de poussières, de meilleure homogénéité en mélange avec d'autres substances...

Cependant, il est extrêmement rare de pouvoir se hasarder à comprimer directement une substance active ou un mélange de substances actives, surtout si celles-ci agissent à des doses très faibles.

Il est donc nécessaire de formuler ces composés, et de faire appel, pour constituer l'excipient, à des adjuvants (encore appelés liants de granulation) de manière à conférer au comprimé final un certain nombre de qualités aussi bien mécaniques que fonctionnelles.

Deux techniques sont habituellement mises en oeuvre pour obtenir un granule : la technique de granulation par voie humide et la technique de granulation par voie sèche.

Dans le cas de la technique de granulation par voie humide, le produit à granuler, soit naturellement humide de par son processus de fabrication en amont, soit artificiellement humidifié par un solvant (eau ou solvant organique), se présente sous une forme pâteuse au moment de la granulation.

Deux procédés sont classiquement mis en oeuvre pour granuler en voie humide : les procédés mécaniques et les procédés physiques.

Les procédés mécaniques consistent en des procédés, bien connus de l'homme du métier, de râpage, de granulation sur rotor ou d'extrusion.

On préfère cependant les procédés de granulation physique qui utilisent une technologie de granulation plus naturelle du produit (et ainsi moins agressive), soit par granulation du produit sur lui-même, soit sur une amorce.

Les générateurs de la granulation proprement dite sont la force centrifuge, la force centripète ou la gravité universelle, associée à un liant de granulation, classiquement constitué d'eau, de solution du produit à granuler, d'alcools ou de vernis...

Le produit à granuler est donc mis en mouvement dans une cuve, par exemple grâce à un flux d'air pulsé, ou sur un disque sphérique ou avec un rotor à lames (suivant le produit et l'effet à obtenir).

Il reçoit en permanence une pulvérisation de liants de granulation sous forme liquide qui assurent l'agglomération.

Ce procédé permet ainsi de réaliser un mélange homogène par contact direct entre le produit à granuler et le liant de granulation. Le mélange intime des deux composants est alors facilité.

Un couteau rotatif horizontal peut effectuer ensuite l'émottage des gros agglomérats.

Dans ce procédé, de loin le plus apprécié par l'homme du métier, il est cependant nécessaire que le liant de granulation, une fois mis en solution, présente une viscosité adéquate, i.e. :
- une viscosité suffisamment faible pour permettre le pompage aisé de la solution renfermant le liant de granulation, pour éviter les problèmes de colmatage en sortie des buses d'injection, pour favoriser la formation de fines gouttelettes et d'en assurer une répartition homogène dans la cuve de granulation,
- une viscosité suffisamment élevée pour permettre au composé de jouer son rôle de liant de granulation.

Quant à la technique de granulation par voie sèche, elle consiste en une opération dite de compactage de la poudre à granuler, cette opération pouvant se faire dans des turbines simples, des compacteurs ou dans des réacteurs sous vide.

Il est fait appel à des procédés de type « frittage » qui conduisent à la fusion superficielle des particules en contact, ce qui donne lieu à une granulation par agglomération.

On fait également appel à des techniques dites « d'agglomération par hybridation », consistant à associer strictement mécaniquement deux particules distinctes de taille différente, en général dans un rapport de 1 à 10, de façon à ce que les petites particules viennent s'incruster en discontinu à la surface de la grande ou constituent un film continu englobant toute la sphère.

Cette technologie est préférée dans le cas où il faut préparer des granules de principes actifs thermosensibles, car il est connu que ce procédé ne génère qu'un échauffement minime, tout en favorisant des réactions mécanochimiques qui permettent d'assurer une bonne cohésion des granules.

Les liants de granulation, en fonction de leur granulométrie initiale, peuvent avoir ce rôle de particules de grande taille qui permettent de fixer des substances actives présentant classiquement une plus fine granulométrie.

L'association directe entre particules de substances à granuler et de liants de granulation est alors favorisée.

L'inconvénient de la technique de granulation par voie sèche est que l'étape ultérieure de calibrage génère une production importante de fines.

La technique de granulation humide présente l'avantage de ne pas faire de fines ou sinon dans des proportions plus faibles.

Cependant, l'adoption de l'une ou de l'autre voie dépend surtout de la nature de la substance active à granuler et, tout particulièrement, de son comportement au cours des opérations de granulation.

Ensuite, les définitions de la voie et du liant de granulation pour granuler ladite substance active permettent d'orienter les qualités physiques et mécaniques du granule obtenu, i.e. son diamètre moyen, sa densité, son aptitude à l'écoulement, son humidité résiduelle et sa friabilité.

De premiers travaux se sont intéressés aux qualités propres de liants de granulation de certaines substances actives, ceci afin de ne pas rendre nécessaire l'ajout de liants de granulation exogènes susceptibles d'altérer la qualité de ladite substance active à granuler (tels les vernis ou les alcools cités ci-avant).

Il est alors procédé à la granulation de la substance active avec un liant de granulation constitué par une solution diluée de cette même substance active.

Par exemple, VELASCO et al. dans Drug Development and Industrial Pharmacy, 21 (10), 1235-1243, 1995, ont décrit les propriétés d'écoulement des poudres de maltodextrines susceptibles d'être utilisées comme vecteur de compression directe.

Selon eux, le paramètre d'écoulement de la poudre de départ est en effet d'importance primordiale pour sa formulation.

Certaines maltodextrines, pour peu qu'elles présentent des propriétés d'écoulement satisfaisantes, peuvent ainsi être avantageusement utilisées pour la granulation d'autres composés, tels les principes actifs d'intérêt pharmaceutique.

VELASCO et al. ont mesuré les paramètres de densité vrac, de compressibilité, d'angle de repos dynamique et surtout d'aptitude à l'écoulement de quatre poudres de maltodextrines commercialisées par la société GRAIN PROCESSING CORP.

VELASCO et al. ont surtout montré que ces maltodextrines présentent des aptitudes à l'écoulement complètement différentes, en fonction du paramètre dit « d'indice de frottement » de la poudre des maltodextrines considérées.

Certaines maltodextrines sont tout à fait satisfaisantes, tandis que d'autres, telles la MALTRIN^{®} M 150, ne passent même pas l'orifice de l'appareil de mesure de l'aptitude à l'écoulement.

Cette grande hétérogénéité de comportement desdites maltodextrines ne favorise donc pas leur utilisation inconditionnelle en granulation, et ne facilite donc que partiellement leur sélection comme liants de granulation.

Les autres substances actives, comme les sucres, les polyols, les édulcorants intenses ou les enzymes, n'ont pas d'aptitudes particulières à la granulation.

Les cristaux de xylitol ou de mannitol ne présentent par exemple aucune aptitude à la compression directe, de même que les édulcorants intenses comme l'aspartame.

Pour ces substances actives particulières, un liant de granulation exogène est donc indispensable pour leur formulation en granules.

Le brevet US 5.583.351 décrit un procédé de préparation d'un produit dense à base d'aspartame sous forme poudre, qui ne présente pas les inconvénients de l'édulcorant de départ, i.e. sa faible dissolution dans l'eau, sa tendance à former des poussières ou sa forte hygroscopicité.

Il ne s'agit pas de granuler l'aspartame en tant que tel, mais de procéder, par des techniques de centrifugation, d'extrusion, de sphéronisation ou d'atomisation, à la granulation de l'aspartame avec un liant constitué de maltodextrines, de dextrines, de gomme arabique, de polyol, de polydextrose ou d'amidon soluble.

Cependant, il est nécessaire d'introduire jusqu'à 40 % de ce liant de granulation. Dans le cas où les maltodextrines sont choisies comme liants de granulation, il est précisé que l'on doit réaliser des mélanges en renfermant de 15 à 25 % en poids.

Un autre inconvénient de ces liants de granulation, est qu'ils n'apportent aucune plus-value nutritionnelle à la substance active à granuler.

Il a alors été proposé de fabriquer des granules de ces substances actives en leur incorporant des fibres alimentaires.

Les effets fibres alimentaires de certains dérivés d'amidon solubles ont alors été développés, ces effets fibres étant la résultante de la combinaison de réactions d'hydrolyse et de réactions de transglucosylation qui confèrent auxdits dérivés d'amidon des propriétés identiques aux fibres diététiques (in ENGLYST et CUMMINGS, American Journal of Clinical Nutrition, 1997, 45, pp 423-431).

C'est ainsi que dans la demande de brevet JP n°2000-37.169, il est décrit des préparations édulcorantes intenses (aspartame, saccharine, sucralose, néotame et leurs dérivés) de faible valeur énergétique, peu visqueuses et ayant des fonctions physiologiques.

Ces nouvelles préparations d'aspartame à teneur en calories réduite sont des granules contenant l'aspartame avec au moins 30 % en poids, voire au moins 50 % en poids de fibres alimentaires apportées par des dextrines indigestibles.

Les procédés de granulation décrits dans ce brevet JP n°2000-37.169 consistent à atomiser une solution aqueuse mixte d'une dextrine indigestible et d'un édulcorant intense ou à créer un noyau de dextrines indigestibles sur lequel on vient vaporiser ledit édulcorant intense.

Cependant, dans ces deux procédés, les dextrines indigestibles doivent être introduites à forte concentration, et il est même recommandé d'introduire lesdites dextrines indigestibles dans des proportions atteignant la quasi-totalité du mélange.

Il est en effet reconnu que ces édulcorants intenses sont des substances actives qui agissent à faible dose, étant donné que leur pouvoir sucrant est jusqu'à 130 à 8000 fois supérieur à celui du saccharose.

Ces dextrines indigestibles sont donc utilisées comme support des substances actives, et non pas comme liants de granulation. Elles ne sont donc destinées qu'à la granulation de substances actives qui agissent en très petites quantités.

Dans la demande de brevet US 2002/0146.487, il est décrit l'enrobage de protéines de soja avec une fine couche d'hydrates de carbone non digestibles, suivi d'une étape de granulation avec de la lécithine.

Cependant, ce procédé a pour objectif la fabrication de granules de protéines de soja aisément dispersibles.

Les protéines de soja présentent en effet déjà d'excellentes aptitudes à la granulation (cf le TOFU qui est une protéine de soja agglomérée).

Le procédé d'enrobage est réalisé de manière à empêcher la pénétration des hydrates de carbone à l'intérieur de l'agglomérat de protéines, et la lécithine est choisie pour ses propriétés surfactantes utilisées classiquement pour favoriser la dispersibilité des protéines.

Aucun de ces deux composants ne sont donc utilisés dans cette demande de brevet comme liants de granulation, bien au contraire.

De tout ce qui précède, il apparaît qu'il n'existe pas de procédé de granulation simple mettant en oeuvre un liant de granulation qui permet d'apporter à la fois une solution aux contraintes technologiques de préparation de granules de substances actives en termes de stabilité mécanique (aptitude à l'écoulement, friabilité, dissolution rapide dans l'eau, comprimabilité) et de conférer aux granules obtenus des propriétés nutritionnelles supplémentaires (par exemple des effets fibres alimentaires), sans qu'il ne soit nécessaire de mettre en oeuvre des quantités importantes dudit liant de granulation. Il est du mérite de la société Demanderesse d'avoir réussi à concilier ces objectifs difficilement conciliables en imaginant et en élaborant, au prix de nombreuses recherches, un procédé simple de préparation de granules de substances actives renfermant des fibres alimentaires.

Le procédé de préparation de granules de substances actives renfermant des fibres alimentaires conforme à l'invention consiste à granuler lesdites substances actives en mélange avec des maltodextrines branchées présentant entre 15 et 35 % de liaisons glucosidiques 1-6, une teneur en sucres réducteurs inférieure à 20 %, un indice de polymolécularité inférieur à 5 et une masse moléculaire moyenne en nombre Mn au plus égale à 4500 g/mole, lesdites maltodextrines branchées constituant entre 3 et 13 % en poids du mélange à granuler.

Ces maltodextrines branchées présentent un caractère d'indigestibilité qui a pour conséquence de diminuer leur pouvoir calorique en empêchant leur assimilation au niveau de l'intestin grêle. Elles constituent donc une source de fibres alimentaires indigestibles.

A titre indicatif, leur taux de fibres insolubles est généralement supérieur à 50 % sur matière sèche, valeur déterminée selon la méthode AOAC n° 985-29 (1986).

Par ailleurs, la faible teneur en molécules de bas degré de polymérisation desdites maltodextrines branchées contribue également à leur caloricité réduite.

Leur teneur élevée en liaisons glucosidiques 1-6 a pour conséquence d'abaisser leur pouvoir cariogène en réduisant leur assimilation par les microorganismes de la cavité buccale.

Ce taux élevé en liaisons 1-6 leur confère également des propriétés prébiotiques tout à fait particulières : il est en effet apparu que les bactéries du cæcum et du colon de l'homme et des animaux, telles que les bactéries butyrogènes, lactiques ou propioniques métabolisent les composés hautement branchés.

D'autre part, ces maltodextrines branchées favorisent le développement des bactéries bifidogènes au détriment des bactéries indésirables. Il en résulte des propriétés tout à fait bénéfiques pour la santé humaine.

La société Demanderesse a trouvé que l'incorporation desdites maltodextrines branchées en mélange avec des substances actives permet de préparer des granules desdites substances actives présentant à la fois d'excellentes propriétés mécaniques et physiques, mais en outre permet de constituer un apport en fibres indigestibles dans des applications auxquelles les liants de granulation classique ne pouvaient prétendre. Toutes les maltodextrines branchées décrites dans la demande de brevet EP 1.006.128 sont appropriées à la préparation des granules de substances actives selon l'invention.

Selon une variante préférée, celles-ci présentent une teneur en sucres réducteurs comprise entre 2 et 5 % et une masse moléculaire en nombre comprise entre 2000 et 3000 g/mole.

Selon une autre variante avantageuse, tout ou partie de ces maltodextrines branchées sont hydrogénées.

Pour la mise en oeuvre du procédé de préparation des granules de substances actives renfermant des fibres alimentaires conforme à l'invention, il est choisi d'incorporer de 3 à 13 % de maltodextrines branchées dans le mélange à granuler.

La société Demanderesse a ainsi vaincu un premier préjugé technique, en choisissant de mettre en oeuvre ces maltodextrines branchées dans des proportions classiques d'un liant de granulation, puisqu'il est établi dans l'état de la technique que des granules renfermant des fibres alimentaires s'entendent de granules renfermant une large proportion desdites fibres, au détriment de la substance active à granuler.

La société Demanderesse a donc établi que le pouvoir de liant de granulation de ses maltodextrines branchées peut s'établir à des doses comprises entre 3 et 13 % en poids du mélange final, de préférence à des doses de 5 % en poids.

Par ailleurs, comme il est connu des spécialistes du domaine technique de la granulation des poudres, il est indispensable que le liant de granulation présente une excellente aptitude à la dispersion en solution et une viscosité adaptées aux contraintes techniques des matériels mis en oeuvre.

La société Demanderesse a alors vaincu un autre préjugé technique relatif à l'utilisation des maltodextrines branchées comme liants de granulation, car comme il sera exemplifié ci-après, un classement relatif des viscosités des fibres solubles disponibles sur le marché indique que les maltodextrines branchées utilisées sont parmi les solutions les plus visqueuses de leur catégorie.

De manière surprenante et inattendue, il a été constaté que cette viscosité ne gêne en aucune façon la mise en forme des substances actives, quel que soit le procédé de granulation choisi.

Dans un premier mode préférentiel de réalisation du procédé conforme à l'invention, les substances actives à granuler sont choisies dans le groupe constitué par les amidons et les dérivés d'amidon.

Dans une première variante, les dérivés d'amidon sont choisis dans le groupe constitué par les dextrines, les dextrines indigestibles, les maltodextrines et les maltodextrines branchées.

La société Demanderesse a en effet constaté que de manière surprenante et inattendue, les maltodextrines branchées utilisées comme liants de granulation permettent de renforcer avantageusement la cohésion des granules constitués d'agents de charge pourtant eux-mêmes classiquement choisis comme liants de granulation.

Comme illustration de cette excellente aptitude à agir comme liants de granulation de ces agents de charge particuliers, la société Demanderesse a réussi à stabiliser le comportement rhéologique des poudres de maltodextrines branchées elles-mêmes, comme il sera exemplifié ci-après.

Dans une deuxième variante, les dérivés d'amidon sont des hydrolysats d'amidon hydrogénés ou des produits de conversion des hydrolysats d'amidon hydrogénés, plus particulièrement des polyols, plus particulièrement encore des polyols choisis dans le groupe constitué par le sorbitol, le mannitol, le xylitol et le maltitol.

Comme illustration de l'aptitude des maltodextrines branchées à agir comme liants de granulation des polyols, des essais ont été entrepris en choisissant deux polyols particulièrement difficiles à granuler, i.e. le xylitol et le mannitol, comme il sera exemplifié ci-après.

Dans un deuxième mode préférentiel de réalisation du procédé conforme à l'invention, les substances actives à granuler sont choisies dans le groupe constitué par les sucres, les édulcorants intenses, les enzymes, les vitamines et les principes actifs pharmaceutiques.

La société Demanderesse a choisi de granuler les substances actives en mettant en oeuvre préférentiellement les techniques de granulation en voie humide.

Un premier mode préférentiel de granulation conforme à l'invention consiste alors à :
- préparer un mélange de substances actives en poudre avec des maltodextrines branchées également en poudre de manière à ce que lesdites maltodextrines branchées représentent entre 3 et 13 %, de préférence environ 5 % en poids sec sur la matière sèche totale du mélange,
- introduire de l'eau à raison de 5 à 20 %, de préférence à raison de 10 % en poids du mélange ainsi préparé de manière à obtenir un mélange homogène de poudres humides,
- agiter mécaniquement le mélange homogène de poudres humides ainsi obtenu dans un mélangeur-granulateur équipé d'une grille de calibrage,
- récupérer et sécher les granules en sortie de ladite grille.

La première étape de ce premier procédé de granulation consiste donc à mélanger la substance active à granuler avec de 3 à 13 %, de préférence de l'ordre de 5 % en poids sec, de maltodextrines branchées.

Cette opération s'effectue par tout moyen connu de l'homme du métier. Il peut être choisi de mélanger les deux composants du mélange dans un mélangeur planétaire de type KENWOOD.

La seconde étape consiste à préparer un mélange homogène de poudres humides. Cette opération s'effectue en introduisant de l'eau, à raison de 5 à 20 %, de préférence à raison de 10 % en poids du mélange.

La troisième étape consiste à granuler ce mélange homogène de poudres humides dans un mélangeur-granulateur équipé d'une grille de calibrage. On peut choisir de réaliser cette étape dans un granulateur en voie humide de type FGS de ERWEKA.

Dans la dernière étape de ce procédé, Les granules obtenus sont alors séchés puis calibrés sur ladite grille de calibrage.

Un deuxième mode préférentiel de granulation conforme à l'invention consiste à :
- préparer une solution des maltodextrines branchées à une matière sèche comprise entre 10 et 50 %, de préférence à une matière sèche d'environ 25 %,
- pulvériser dans un séchoir-granulateur la solution de maltodextrines branchées ainsi obtenue sur la poudre de substances actives, les maltodextrines branchées représentant entre 3 et 13 %, de préférence environ 5 % en poids sec de la matière sèche totale du mélange,
- récupérer et sécher les granules ainsi obtenus.

La première étape de ce deuxième procédé de granulation consiste donc à préparer tout d'abord une solution de maltodextrines branchées, à une matière sèche comprise entre 10 et 50 %, de préférence à une matière sèche de 25 %.

Les propriétés naturelles de dissolution rapide des maltodextrines branchées, supérieures à celles des maltodextrines équivalentes standards, favorisent leur mise en solution à une telle matière sèche.

La deuxième étape consiste à pulvériser la solution de maltodextrines branchées ainsi obtenue sur de la poudre de substances actives, les maltodextrines branchées représentant entre 3 et 13 %, de préférence 5 % en poids sec de la matière sèche totale du mélange. Cette opération peut être avantageusement réalisée dans un séchoir granulateur à lit d'air fluidisé de type STREA-1 de AEROMATIC muni d'une buse d'injection.

La société Demanderesse a ainsi pu constater que malgré la viscosité dynamique relative de la solution de maltodextrines préparée, il n'est déploré aucun problème :
- de pompage de la solution de ce liant de granulation,
- de pulvérisation à la sortie des buses d'injection du liquide dans la masse de particules de substances actives en mouvement dans le granulateur mélangeur,
- d'hétérogénéité du mélange produit,
- de disparité dans la répartition des gouttelettes du liant de granulation projeté.

La troisième étape de ce procédé consiste enfin à récupérer et sécher les granules ainsi obtenus, par tout moyen connu de l'homme du métier.

Cette aptitude à granuler facilement ces différentes substances actives conduit tout naturellement à utiliser et à proposer les maltodextrines branchées présentant entre 15 et 35 % de liaisons glucosidiques 1-6, une teneur en sucres réducteurs inférieure à 20 %, un indice de polymolécularité inférieur à 5 et une masse moléculaire moyenne en nombre Mn au plus égale à 4500 g/mole comme liant de granulation pour la préparation de granules de substances actives.

D'autres caractéristiques et avantages de la présente invention apparaîtront clairement à la lecture des exemples donnés ci-après qui viennent illustrer l'invention sans toutefois la limiter.

### Exemple 1

La détermination de la viscosité relative des solutions de fibres solubles disponibles du marché, susceptibles d'apporter comme liant de granulation une composante nutritionnelle aux granules de substances actives, a été réalisée de la manière suivante.

La viscosité de ces différents produits a été mesurée au moyen d'un rhéomètre rotatif à déformation imposée ARES (RHEOMETRIC SCIENTIFIC) équipé d'une géométrie de type cône-plan. Un gradient de cisaillement est appliqué au produit, et la contrainte (couple de rotation) nécessaire au cisaillement est mesurée. Les mesures de viscosités (Eta) sont exprimées en Pa.s par le rapport entre la contrainte et le gradient de cisaillement. Le système de mesure est thermostaté, et la température est maintenue à +/- 0,1°C.

On prépare des solutions à une matière sèche de 25 % de chacune des fibres commerciales, et on réalise la mesure de la viscosité desdites solutions à la température de 20°C.

Le tableau I suivant présente la viscosité relative des maltodextrines branchées choisies comme liants de granulation selon l'invention, en regard du polydextrose, des dextrines indigestibles, des fructo-oligosaccharides et des galacto-oligosaccharides disponibles du commerce.

L'indice de viscosité a été fixé à 1 pour les maltodextrines branchées présentant une teneur en sucres réducteurs comprise entre 2 et 5 % et une masse moléculaire en nombre comprise entre 2000 et 3000 g/mole utilisées dans l'invention.

**Tableau I. Classement relatif des viscosités des fibres solubles du marché**

| Nom de la fibre soluble | Fabricant | Type de molécule | Indice de viscosité |
|---|---|---|---|
| LITESSE II | CULTOR | Polydextrose | 0,6 |
| PINE FIBER | MATSUTANI | Dextrine indigestible | 0,4 |
| LITESSE I | CULTOR | Polydextrose | 0,3 |
| LITESSE III | CULTOR | Polydextrose | 0,2 |
| ACTILIGHT 950 P | BEGHIN MEIJI | Fructo-oligosaccharide | 0,2 |
| CUP OLIGO P | NISSHIN SEITO | Galacto-oligosaccharide | 0,2 |
| RAFTILOSE P95 | ORAFTI | Fructo-oligosaccharide | 0,2 |

Il apparaît clairement que les maltodextrines branchées utilisées ici présentent l'indice de viscosité le plus élevé de leur catégorie, ce qui ne les destine donc naturellement pas à être mises en oeuvre comme liants de granulation.

### Exemple 2

Pour illustrer la capacité des maltodextrines branchées à pouvoir agir comme liants de granulation, on réalise la granulation sur elles-mêmes desdites maltodextrines branchées décrites dans l'exemple 1.

Cette capacité des maltodextrines branchées d'agir comme liant de granulation doit permettre d'améliorer leurs propriétés physiques et mécaniques, voire de gommer les défauts de la poudre de maltodextrines branchées de départ, notamment en termes de production de poussières.

On prépare une solution de maltodextrines branchées à 25 % de matière sèche (25 g de maltodextrines branchées avec 75 g d'eau).

On dépose 475 g d'une poudre de maltodextrines branchées d'une taille moyenne de particules de 77 µm dans le bol du séchoir granulateur à lit d'air fluidisé de type STREA-1 de AEROMATIC muni d'une buse d'injection.

Par de l'air pulsé à la base dudit bol, on met en suspension la poudre à une température de 60°C. On pulvérise ensuite la solution de maltodextrines branchées à un débit de 4 ml/min et à une pression de 1 bar.

Les granules récupérés après 25 à 30 min de temps de séjour, sont récupérés et séchés dans ledit granulateur pendant 30 minutes à 60°C. Les granules sont ensuite calibrés sur un tamis de 1250 µm de taille de maille.

On mesure ensuite la granulométrie, l'aptitude à l'écoulement, la densité aérée et la comprimabilité des granules obtenus. On réalise également un test de poussières afin d'appréhender la cohésion desdits granules.

La mesure de la taille des granules est effectuée ici sur granulomètre laser LS de marque COULTER^{®}, exprimée par la moyenne arithmétique des tailles des particules (µm) obtenues.

L'écoulement des granules est déterminé par un test « entonnoir » selon la méthode de pharmacotechnie 2.9.16 de la Pharmacopée Européenne, 3^{ème} édition, et qui consiste à mesurer la vitesse d'écoulement, exprimée en secondes, de 100 g de granules déposés dans un entonnoir dont les dimensions sont données précisément dans cette méthode.

La densité aérée est quant à elle mesurée par un test à l'éprouvette, qui consiste à utiliser une éprouvette de 250 ml, graduée tous les 2 ml. 100 g de granules pesés avec une précision de 0,5 % sont introduits sans tasser dans l'éprouvette sèche. On lit ensuite le volume apparent non tassé Vo estimé à 1 ml près.

La densité aérée mesurée ici correspond au rapport du poids de granules (ici 100 g) sur le volume mesuré à l'éprouvette (Vo).

La mesure de comprimabilité consiste à déterminer la force, exprimée en Newton, qui correspond à la résistance à l'écrasement de comprimés mesurée sur duromètre SCHLEUNIGER 2E.

Ces comprimés, produits sur presse alternative FROGERAIS / SVIAC équipée de poinçons concaves de diamètre 13 mm, à partir d'un mélange de 99,5 % de granules conformes à l'invention et de 0,5 % de stéarate de magnésium sont de forme cylindrique à faces convexes (rayon de courbure de 13 mm), d'un diamètre de 13 mm, d'une épaisseur de 6 mm, et de poids égal à 0,771 g, soit d'un volume de 0,571 cm³, et d'une masse volumique apparente de 1,35 g/cm³.

Le test de poussières consiste enfin à mesurer l'aptitude d'un granule à libérer de fines particules dans un flux d'air calibré. Il est utilisé pour cette mesure un appareil HEUBACH-DUSTMETER monté dans sa configuration de type II. 100 g de granules sont introduits dans l'appareil, et soumis à un flux d'air à un débit de 8 l/min pendant 5 min, à une température de 20°C.

Les fines particules entraînées par ce flux d'air sont recueillies sur un filtre papier qui est ensuite pesé. Le test de poussières consiste donc à déterminer le poids de fines particules déposées à la surface du filtre.

Le tableau II suivant présente les résultats obtenus sur les granules de maltodextrines branchées conformes à l'invention (granules A), en comparaison de la poudre de maltodextrines branchées de départ. En témoin, figurent les résultats des analyses effectuées sur des granules (granules B) obtenus en prenant comme liant de granulation une maltodextrine particulière, commercialisée par la société Demanderesse sous le nom de marque LYCATAB^{®} DSH comme excipient de granulation en voie humide, à la place des maltodextrines branchées et en procédant exactement de la même façon que pour obtenir les granules A.

**Tableau II. Résultats d'analyses des granules obtenus**

| | Maltodextrines branchées de départ | Granules A | Granules B |
|---|---|---|---|
| Granulométrie (µm) | 77 | 160 | 136,5 |
| Ecoulement des poudres(s) | 8 | 9 | 10 |
| Masse volumique apparente(g/ml) | 0,375 | 0,393 | 0,387 |
| Comprimabilité (N) | 250 | 190 | 195 |
| Test de poussières (g) | 0,0925 | 0,0221 | 0,0325 |

La granulation des maltodextrines branchées sur elles-mêmes permet de réduire remarquablement les poussières générées par la manipulation des poudres de maltodextrines branchées de départ, tout en permettant d'obtenir des granules dont l'aptitude à l'écoulement, la densité et la comprimabilité sont conformes à ce que l'on est en droit de s'attendre d'un liant de granulation efficace, puisque les valeurs sont équivalentes à celles obtenues avec le LYCATAB^{®} DSH.

### Exemple 3

On réalise la granulation de xylitol cristallin commercialisé par la société Demanderesse sous le nom de marque XYLISORB^{®} 300 (granulométrie moyenne de 175,8 µm) avec les maltodextrines branchées de l'exemple 1, dans les mêmes conditions que celles décrites dans l'exemple 2.

Le tableau III suivant présente les résultats des analyses effectuées sur les granules de xylitol préparés avec les maltodextrines branchées comme liants de granulation (granules C), en comparaison avec la poudre de xylitol de départ.

En témoin, figurent les résultats des analyses réalisées sur des granules obtenus en prenant également le LYCATAB^{®} DSH comme liant de granulation en voie humide, à la place des maltodextrines branchées (granules D).

**Tableau III. Résultats d'analyses des granules obtenus**

| | XYLISORB^{®} 300 de départ | Granules C | Granules D |
|---|---|---|---|
| Granulométrie (µm) | 175,8 | 229 | 256 |
| Ecoulement des poudres (s) | Infini | 11 | 13 |
| Masse volumique apparente (g/ml) | 0,583 | 0,578 | 0,521 |
| Comprimabilité (N) | ND* | 130 | 120 |
| Test de poussières (g) | 0 | 0,0014 | 0,0024 |

| | | | |
|---|---|---|---|
| ND* : Non Détectable | | | |

La granulation du xylitol à l'aide des maltodextrines branchées comme liants de granulation, tout comme la granulation réalisée avec le LYCATAB^{®} DSH, permettent de conférer au xylitol des propriétés d'écoulement qu'il ne possède absolument pas. Cette granulation est par ailleurs la seule façon de conférer au xylitol une comprimabilité satisfaisante.

Il est cependant à noter, qu'en plus de leur apport supplémentaire en fibres alimentaires, les maltodextrines branchées utilisées comme liants de granulation permettent d'améliorer l'écoulement des granules de xylitol et de limiter un peu plus la formation de poussières, en regard du LYCATAB^{®} DSH pris comme liant de granulation classique.

### Exemple 4

On réalise la granulation de mannitol cristallin avec les maltodextrines branchées de l'exemple 1.

On mélange 950 g de mannitol cristallin commercialisé par la société Demanderesse sous l'appellation MANNITOL P60 (granulométrie moyenne de 60 µm) avec 50 g d'une poudre de maltodextrines branchées dans le mélangeur planétaire de type KENWOOD à vitesse minimale pendant 5 minutes.

On ajoute de l'eau à raison de 10 parts d'eau pour 100 parts du mélange ainsi obtenu, et on continue à mélanger pendant 10 minutes.

La granulation est ensuite réalisée sur un granulateur voie humide type FGS de ERWEKA équipé d'une grille de 1000 µm, en suivant les spécifications du constructeur.

Les granules obtenus sont ensuite séchés dans un séchoir en lit d'air fluidisé de laboratoire AEROMATIC de type STREA 1 à une température de 60°C pendant 30 min. Les granules sont ensuite calibrés sur ladite grille de calibrage de 1000 µm (granules E).

On réalise en témoin, dans les mêmes conditions opératoires, une granulation du MANNITOL P60 avec 5 % de LYCATAB^{®} DSH comme excipient de granulation humide (granules F).

Les résultats des mesures comparatives des granules obtenus avec ces deux liants de granulation en termes de densité de poudre, de temps d'écoulement et de comprimabilité, effectuées dans des conditions identiques aux exemples 2 et 3, sont figurés dans le tableau IV suivant.

Les mesures de granulométrie sont ici faites sur tamiseuse électromagnétique de laboratoire FRITSH de type ANALYSETTE 3, et sont exprimées en diamètre moyen des particules (taille des 50 % de particules totales).

**Tableau IV. Résultats d'analyses des granules obtenus**

| | Mannitol P60 de départ | Granules E | Granules F |
|---|---|---|---|
| Diamètre moyen (µm) | 60 | 860 | 860 |
| Ecoulement des poudres (s) | Infini | 13 | 13 |
| Masse volumique apparente (g/ml) | 0,588 | 0,540 | 0,550 |
| Comprimabilité (N) | ND* | 80 | 80 |

La granulation du mannitol cristallin avec les maltodextrines branchées comme liants de granulation permet d'obtenir des granules dont l'aptitude à l'écoulement, la densité et la comprimabilité sont conformes à ce que l'on est en droit de s'attendre d'un liant de granulation efficace, puisque les valeurs sont équivalentes à celles obtenues avec le LYCATAB^{®} DSH. La nature intrinsèque des maltodextrines branchées en tant que fibres alimentaires peut ainsi être pleinement valorisée en plus de leurs propriétés en tant que liants de granulation.

## Revendications

1. Procédé de préparation de granules de substances actives renfermant des fibres alimentaires, **caractérisé par le fait qu'**il consiste à granuler lesdites substances actives en mélange avec des maltodextrines branchées présentant entre 15 et 35 % de liaisons glucosidiques 1-6, une teneur en sucres réducteurs inférieure à 20 %, un indice de polymolécularité inférieur à 5 et une masse moléculaire moyenne en nombre Mn au plus égale à 4500 g/mole, lesdites maltodextrines branchées constituant entre 3 et 13 % en poids du mélange à granuler.

2. Procédé selon la revendication 1, **caractérisé par le fait que** les substances actives sont choisies dans le groupe constitué par les amidons et les dérivés d'amidon.

3. Procédé selon la revendication 2, **caractérisé par le fait que** les dérivés d'amidon sont choisis dans le groupe constitué par les dextrines, les dextrines indigestibles, les maltodextrines et les maltodextrines branchées.

4. Procédé selon la revendication 2, **caractérisé par le fait que** les dérivés d'amidon sont des hydrolysats d'amidon hydrogénés ou des produits de conversion des hydrolysats d'amidon hydrogénés, plus particulièrement des polyols, plus particulièrement encore des polyols choisis dans le groupe constitué par le sorbitol, le mannitol, le xylitol et le maltitol.

5. Procédé selon la revendication 1, **caractérisé par le fait que** lesdites substances actives sont choisies dans le groupe constitué par les sucres, les édulcorants intenses, les enzymes, les vitamines et les principes actifs pharmaceutiques.

6. Procédé selon l'une quelconque des revendications 1 à 5, **caractérisé par le fait qu'**il consiste à :
- préparer un mélange de substances actives en poudre avec des maltodextrines branchées en poudre de manière à ce que lesdites maltodextrines branchées représentent entre 3 et 13 %, de préférence environ 5 % en poids sec sur la matière sèche totale du mélange,
- introduire de l'eau à raison de 5 à 20 %, de préférence à raison de 10 % en poids du mélange ainsi préparé, de manière à obtenir un mélange homogène de poudres humides,
- agiter mécaniquement le mélange homogène de poudres humides ainsi obtenu dans un mélangeur-granulateur équipé d'une grille de calibrage,
- récupérer et sécher les granules en sortie de ladite grille.

7. Procédé selon l'une quelconque des revendications 1 à 5, **caractérisé par le fait qu'**il consiste à :
- préparer une solution de maltodextrines branchées à une matière sèche comprise entre 10 et 50 %, de préférence à une matière sèche d'environ 25 %,
- pulvériser dans un séchoir-granulateur la solution de maltodextrines branchées ainsi obtenue sur la poudre de substances actives, les maltodextrines branchées représentant entre 3 et 13 %, de préférence environ 5 % en poids sec de la matière sèche totale du mélange,
- récupérer et sécher les granules ainsi obtenus.

8. Procédé selon l'une quelconque des revendications 1 à 7, **caractérisé par le fait que** les maltodextrines présentant entre 15 et 35 % de liaisons glucosidiques 1-6, une teneur en sucres réducteurs inférieure à 20 %, un indice de polymolécularité inférieur à 5 et une masse moléculaire moyenne en nombre Mn au plus égale à 4500 g/mole, sont utilisés comme liant de granulation de substances actives.

9. Utilisation des maltodextrines présentant entre 15 et 35 % de liaisons glucosidiques 1-6, une teneur en sucres réducteurs inférieure à 20 %, un indice de polymolécularité inférieur à 5 et une masse moléculaire moyenne en nombre Mn au plus égale à 4500 g/mole, comme liant de granulation de substances actives.

## Claims

1. A method for preparing granules of active substances containing dietary fibres, **characterized in that** it consists in granulating a mixture of said active substances and branched maltodextrins having between 15 and 35% of 1-6 glucoside linkages, a reducing sugar content of less than 20%, a polymolecularity index of less than 5 and a number-average molecular mass Mn at most equal to 4500 g/mol, said branched maltodextrins content being of between 3 and 13% by weight of the mixture to be granulated.

2. The method as claimed in claim 1, **characterized in that** the active substances are selected from the group consisting of starches and starch derivatives.

3. The method as claimed in claim 2, **characterized in that** the starch derivatives are selected from the group consisting of dextrins, indigestible dextrins, maltodextrins and branched maltodextrins.

4. The method as claimed in claim 2, **characterized in that** the starch derivatives are hydrogenated starch hydrolysates or conversion products of the hydrogenated starch hydrolysates, more particularly polyols, even more particularly polyols selected from the group consisting of sorbitol, mannitol, xylitol and maltitol.

5. The method as claimed in claim 1, **characterized in that** said active substances are selected from the group consisting of sugars, strong sweeteners, enzymes, vitamins and pharmaceutical active principles.

6. The method as claimed in any one of claims 1 to 5, **characterized in that** it consists in
- preparing a mixture of powdered active substances with powdered branched maltodextrins such that said branched maltodextrins content is of between 3 and 13%, preferably approximately 5%, by dry weight relative to the total solids content of the mixture,
- introducing water in a proportion of 5 to 20%, preferably in a proportion of 10%, by weight of the resulting mixture, so as to obtain a homogeneous mixture of wet powders,
- mechanically agitating the resulting homogeneous mixture of wet powders, in a mixer-granulator equipped with a sizing screen,
- recovering and drying the granules as they exit said screen.

7. The method as claimed in any one of claims 1 to 5, **characterized in that** it consists in
- preparing a solution of branched maltodextrins at a solids content of between 10 and 50%, preferably at a solids content of approximately 25%,
- spraying the resulting solution of branched maltodextrins onto the powder of active substances, in a dryer-granulator, the branched maltodextrins content is of between 3 and 13% preferably approximately 5%, by dry weight of the total solids content of the mixture,
- recovering and drying the resulting granules.

8. The method as claimed in any one of claims 1 to 7, **characterized in that** the maltodextrins having between 15 and 35% of 1-6 glucoside linkages, a reducing sugar content of less than 20%, a polymolecularitry index of less than 5 and a number-average molecular mass Mn at most equal to 4500 g/mol are used as a granulation binder for active substances.

9. The use of maltodextrins having between 15 and 35% of 1-6 glucoside linkages, a reducing sugar content of less than 20%, a polymolecularity index of less than 5 and a number-average molecular mass Mn at most equal to 4500 g/mol, as a granulation binder for active substances.

## Patentansprüche

1. Verfahren zur Herstellung von Wirkstoffkörnern, enthaltend Ballaststoffe, **dadurch gekennzeichnet, dass** es aus dem Granulieren dieser Wirkstoffe in einer Mischung mit verzweigten Maltodextrinen besteht, die zwischen 15 und 35 % 1-6 glucosidischer Bindungen, einen Gehalt an reduzierten Zuckern unter 20 %, einen Polymolekularitätsindex unter 5 und ein zahlengemitteltes Molekulargewicht Mw von höchstens gleich 4.500 g/mol aufweisen, wobei die verzweigten Maltodextrine zwischen 3 und 13 Gew.% des zu granulierenden Gemisches ausmachen.

2. Verfahren gemäss Anspruch 1, **dadurch gekennzeichnet, dass** die Wirkstoffe aus der Gruppe, bestehend aus Stärken und Stärkederivaten, ausgewählt sind.

3. Verfahren gemäss Anspruch 2, **dadurch gekennzeichnet, dass** die Stärkederivate aus der Gruppe, bestehend aus Dextrinen, unverdaulichen Dextrinen, Maltodextrinen und verzweigten Maltodextrinen, ausgewählt sind.

4. Verfahren gemäss Anspruch 2, **dadurch gekennzeichnet, dass** die Stärkederivate Hydrolysate hydrierter Stärke oder umgewandelte Produkte von Hydrolysaten hydrierter Stärke sind, bevorzugt von Polyolen, mehr bevorzugt Polyolen, ausgewählt aus der Gruppe bestehend aus Sorbit, Mannit, Xylit und Maltit.

5. Verfahren gemäss Anspruch 1, **dadurch gekennzeichnet, dass** die Wirkstoffe ausgewählt werden aus der Gruppe, bestehend aus Zuckern, Süssstoffen, Enzymen, Vitaminen und pharmazeutischen Wirkstoffen.

6. Verfahren gemäss irgendeinem der Ansprüche 1 bis 5, **dadurch gekennzeichnet, dass** es besteht aus:
• Herstellen einer Mischung aus Wirkstoffen in Pulverform mit verzweigten Maltodextrinen in Pulverform, wobei die verzweigten Maltodextrine zwischen 3 und 13 % ausmachen, bevorzugt etwa 5 Gew.% des Trockengewichts der gesamten getrockneten Materie des Gemisches,
• Hinzufügen von Wasser in einem Verhältnis von 5 bis 20 %, bevorzugt in einer Menge von 10 Gew.% des Gemisches, das so hergestellt wurde, um ein homogenes Gemisch angefeuchteter Pulver zu erhalten,
• mechanisches Mischen des homogenen Gemisches der angefeuchteten Pulver, die auf diese Weise gewonnen wurden, in einem Mischer-Granulator, der mit einem Kalibrierungsgitter ausgerüstet ist,
• Einsammeln und Trocknen der Körner, die aus diesem Gitter austreten.

7. Verfahren gemäss irgendeinem der Ansprüche 1 bis 5, **dadurch gekennzeichnet, dass** es besteht aus:
• Herstellen einer Lösung verzeigter Maltodextrine mit einem Trockengewicht zwischen 10 und 50 %, bevorzugt mit einem Trockengewicht von etwa 25 %,
• Pulverisieren der Lösung aus verzweigten Maltodextrinen, die auf diese Weise gewonnen wurde, auf dem Wirkstoffpulver in einem Trockner-Granulator, wobei die verzweigten Maltodextrine zwischen 3 und 13 % ausmachen, bevorzugt etwa 5 Gew.% Trockengewicht des Gesamttrockengewichts der Mischung,
• Einsammeln und Trocknen der so erhaltenen Körner.

8. Verfahren gemäss irgendeinem der Ansprüche 1 bis 7, **dadurch gekennzeichnet, dass** die Maltodextrine, welche zwischen 15 und 35 % 1-6 glucosidischer Bindungen, einen Gehalt reduzierter Zucker unter 20 %, einen Polymolekularitätsindex unter 5 und ein zahlengemitteltes Molekulargewicht Mw von höchstens gleich 4.500 g/mol aufweisen, als Bindemittel bei der Granulierung wirksamer Substanzen verwendet werden.

9. Verwendung von Maltodextrinen, die zwischen 15 und 35 % 1-6 glucosidischer Bindungen, einen Gehalt an reduzierten Zuckern unter 20 %, einen Polymolekularitätsindex unter 5 und ein zahlengemitteltes Molekulargewicht Mw von höchstens 4.500 g/mol aufweisen, als Bindemittel zur Granulierung von Wirkstoffen.
